# EUROPEAN PATENT APPLICATION

(11) **EP 4 309 675 A1**
(43) Date of publication of application: **24.01.2024**
(21) Application number: 21931034.9
(22) Date of filing: 08.06.2021
(51) Int. Cl.: A61K 47/54, A61K 31/635, A61K 9/08, A61P 27/02, A61P 29/00

(54) **PRODRUG OF CELECOXIB, PREPARATION METHOD THEREFOR AND APPLICATION THEREOF**

(30) Priority: 15.03.2021 CN 202110276245
(71) Applicant: Guangzhou Ocusun Ophthalmic Biotechnology Co., Ltd., Guangzhou, Guangdong 511400 (CN)
(72) Inventor: WANG, Yandong, Guangzhou1, Guangdong 510600 (CN); LIU, Guoqiang, Shijiazhuang, Hebei 050000 (CN); LIU, Wei, Shanghai 210516 (CN)
(74) Representative: Latscha Schöllhorn Partner AG
(86) International application number: PCT/CN2021/098759
(87) International publication number: WO 2022/193449

(57) **Abstract**

Disclosed are a prodrug of celecoxib represented by Formula I, a preparation method therefor and an application thereof. The compound has proper solubility and is relatively stable under storage conditions. Pharmacokinetic experiments show that celecoxib can be detected in ocular tissues and is distributed in cornea, conjunctiva and aqueous humor, and the celecoxib achieves a relatively high drug concentration and thus the compound of Formula I is an excellent celecoxib prodrug. Moreover, the distribution of control compound SZY1907-P4 is almost not detected, and the concentration of eye drops prepared from the celecoxib in the cornea, conjunctiva and aqueous humor is also relatively low. The compound of Formula I has a remarkable absorption advantage, the drug concentration in the ocular tissues is remarkably higher than those of the celecoxib group and control group SZY1907-P4, and thus the compound can be prepared into liquid preparations, such as eye drops and injections, for inflammatory reactions.

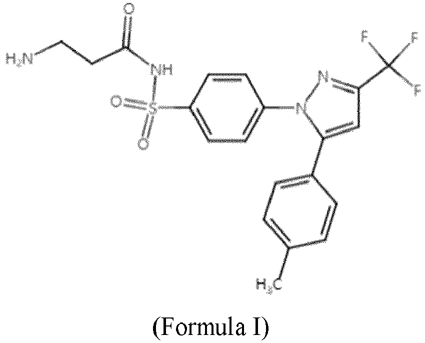

## Description

### TECHNICAL FIELD

The present invention belongs to the field of medicine, in particular relates to a prodrug of celecoxib, preparation method therefor and application thereof.

### BACKGROUND

Celecoxib is an anti-inflammatory and analgesic drug that selectively inhibits cyclooxygenase-2, which can inhibit the production of prostaglandins, and reduce edema and pain of inflammation. In clinic, it is mainly used for treating joint pain and limited movement caused by knee osteoarthritis. It also has a good therapeutic effect on rheumatism and rheumatoid arthritis. In addition, it can also effectively relieve acute pain such as caused by trauma or surgery, and chronic pain such as caused by lumbar muscle strain, cervical type cervical spondylosis, or internal and external humeral epicondylitis. An eye drops made directly from celecoxib has a lower concentration of celecoxib in the eye tissue.

### SUMMARY OF THE INVENTION

The object of the present invention is to provide a prodrug of celecoxib and a pharmaceutically acceptable salt thereof.

The structure of the prodrug of celecoxib is as shown in Formula I:

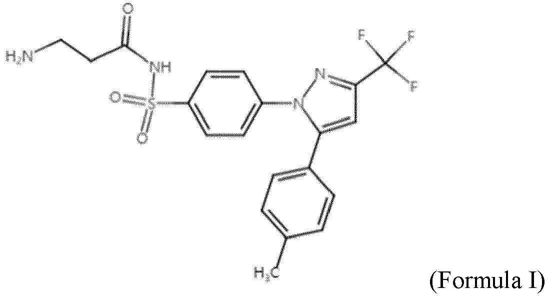

The pharmaceutically acceptable salt of compound of Formula I refers to a salt that, within the scope of reliable medical judgment, is suitable for use in contact with tissues of human and lower animal without excessive toxicity, irritation, allergic reactions, and the like, and commensurate with a reasonable effect/risk ratio. The pharmaceutically acceptable salts of the compound of Formula I include but are not limited to citrate, trans-butene dioic acid salt, salicylate, L-tartrate, fumarate, sodium salt, potassium salt, calcium salt, hydrochloride, acetate, nitrate, sulfate, bisulfate, phosphate, hydrophosphate, acetate, oxalate, lactate, lysinate, aspartate and the like.

The present invention further provides a method for the preparation of the above-mentioned compound of Formula I.

The method for the preparation of the compound of Formula I according to the present invention comprises the following steps:
1) the compound of Formula II (celecoxib) and the compound of Formula III (Boc-β- alanine) are reacted in the presence of EDCI/ DMAP/ triethylamine, to obtain the compound of Formula IV (SZY1907-05); and 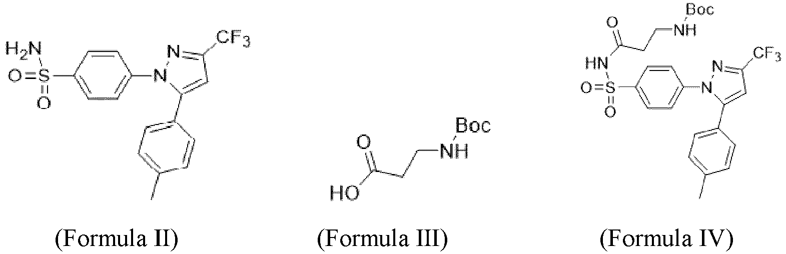
2) the compound of Formula IV is reacted with a solution of hydrogen chloride in ethyl acetate, to obtain the compound of Formula I.

In step 1) of the above method, the molar ratio of the compound of Formula II to the compound of Formula III is 1:1.4.

In step 1) of the above method, the molar ratio of the compound of Formula II to EDCI, DMAP and triethylamine is 2.3: 0.32: 3, respectively.

In step 1) of the above method, the reaction is performed in a solvent, wherein the solvent is specifically DCM (dichloromethane).

In step 1) of the above method, the reaction is performed under stirring conditions at room temperature and monitored by TLC until there is no more raw material.

In step 1) of the above method, the method further comprises the following post-treatment steps after the reaction is finished: washing once with 3% diluted hydrochloric acid, washing once with saturated ammonium chloride, washing with saturated sodium chloride to a neutral condition, drying the organic phase with sodium sulfate, rotary drying to obtain a white solid; dissolving the white solid in ethanol, adding an equivalent amount of water, heating the temperature to reflux until the white solid being completely dissolved, slowly cooling to precipitate a product, vacuum filtration and drying to obtain SZY1907-05.

In step 2) of the above method, the molar ratio of the compound of Formula IV to hydrogen chloride is 0.05: 1.71.

In step 2) of the above method, the reaction condition is stirring at room temperature; a solid is precipitated gradually during the reaction process, and the reaction is monitored by TLC until there is no more raw material.

In step 2) of the above method, the method further comprises the following post-treatment steps after the reaction is finished: rotary drying, drip washing the solid with ethyl acetate, collecting the filter cake and rotary drying to a constant weight to obtain the compound of Formula I.

Another object of the present invention is to provide a use of the compound of Formula I.

The present invention provides the use of the compound of Formula I or a pharmaceutically acceptable salt thereof in the preparation of a medicament for the prevention and/or treatment of inflammation of an ocular tissue.

The medicament prepared using the compound of Formula I as an active ingredient for the prevention and/or treatment of inflammation of an ocular tissue also belongs to the protection scope of the present invention.

The medicament may be introduced for example into muscle, intradermal, subcutaneous, venous, or mucosal tissue of a body by injection, spray, nasal drop, eye drop, penetration, absorption, physical or chemical mediated methods; or it is mixed or encapsulated by other substances and then introduced into the body.

If necessary, one or more pharmaceutically acceptable carriers may also be added to the above medicament. Pharmaceutically acceptable carriers include a conventional diluent, excipient, filler, adhesive, humectant, disintegrant, sorbefacient, surfactant, adsorption carrier, lubricant, and the like.

The present invention further provides a medicament or a pharmaceutical composition for the prevention and/or treatment of inflammation of an ocular tissue, the active ingredient thereof comprises the compound of Formula I or a pharmaceutically acceptable salt thereof.

Such medicament or pharmaceutical composition may be prepared as a liquid formulation, such as eye drops, injection or other forms, in accordance with conventional methods known to those skilled in the art.

The present invention further provides an eye drops for the prevention and/or treatment of inflammation of an ocular tissue.

The eye drops according to the present invention contain the following constituents per 100 mL of the eye drops: 0.1 g of the compound of Formula I, 0.05 g of Tween-80, 0.45 g of polyoxyethylene 40 hydrogenated castor oil, 0.9 g of sodium chloride, 0.01 g of benzalkonium chloride, and the balance being distilled water; and the pH value of the eye drops is 5.5-6.5.

The compound of Formula I according to the present invention has proper solubility and is relatively stable under storage conditions. Pharmacokinetic experiments show that celecoxib can be detected in ocular tissues and is distributed in cornea, conjunctiva and aqueous humor, and celecoxib achieves a relatively high drug concentration and thus the compound of Formula I is an excellent celecoxib prodrug. Moreover, the distribution of the control compound SZY1907-P4 is almost not detected, and the concentration of eye drops prepared from celecoxib in cornea, conjunctiva and aqueous humor is also relatively low. The compound of Formula I has a remarkable absorption advantage, the drug concentration in the ocular tissues is remarkably higher than those of the celecoxib group and control group SZY1907-P4, and thus the compound can be prepared as liquid preparations, such as eye drops and injections, for inflammatory reactions.

### DESCRIPTION OF THE DRAWINGS

Figure 1 shows the HNMR spectrum of SZY1907-P3.
Figure 2 shows the LC-MS spectrum of SZY1907-P3.

### DETAILED DESCRIPTION OF THE EMBODIMENTS

The present invention is further described in combination with specific examples below, but the present invention is not limited to the following examples. The methods described are conventional unless otherwise specified. The raw materials are commercially available unless otherwise specified.

### Example 1. Preparation of compound of Formula I SZY1907-P3

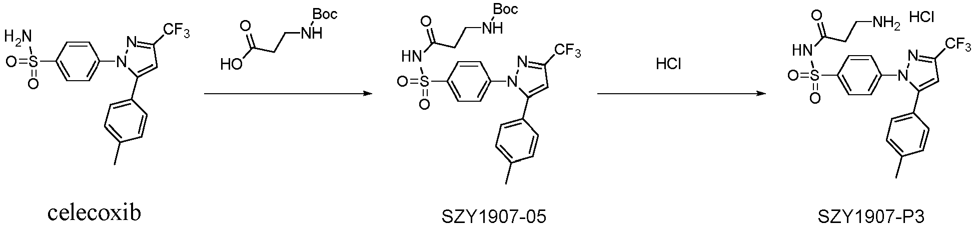

### Preparation of SZY1907-05:

To a three-neck flask added celecoxib (20.00 g, 0.0525 mol) and DCM (400 mL) , then Boc-β-alanine (0.0735 mol), EDCI (0.121 mol), triethylamine (0.158 mol), DMAP (0.0164 mol) was added successively. The reaction was stirred at room temperature, and monitored by TLC until no more raw materials were detected.

Post-treatment: washing once with 3% diluted hydrochloric acid, washing once with saturated ammonium chloride, washing with saturated sodium chloride to a neutral condition, drying the organic phase with sodium sulfate, rotary drying to obtain a white solid; dissolving the white solid in ethanol, adding an equivalent amount of water, raising the temperature to reflux until the white solid being completely dissolved, slowly cooled to precipitate a product, vacuum filtration and drying to obtain SZY1907-05, with the yield of about 95%, and the purity of greater than 98%.

State: White solid.

### Preparation of SZY1907-P3:

To a three-neck flask added SZY1907-05 (28.00 g, 0.05 mol), and then a solution of hydrogen chloride in ethyl acetate (3.8 mol/L, 450 mL) was added, a solid gradually precipitated during the reaction process, which was monitored by TLC until no more raw materials were detected.

Post-treatment: rotary drying, drip washing the solid with 200 mL of ethyl acetate, collecting the filter cake and rotary drying to a constant weight to obtain the product, with the yield of about 85%, and the purity of greater than 99%.

State: White solid.

¹HNMR H₂O δ: 7.55-7.53 (d, J=7.6 Hz, 2H), 6.83-6.81 (d, J=6.8 Hz, 2H), 6.54-6.46 (m, 4H), 6.15 (s, 1H), 3.05-3.04 (m, 2H), 2.64 (m, 2H), 1.76 (m, 3H).

LC-MS: m/z = 453.5(M+1).

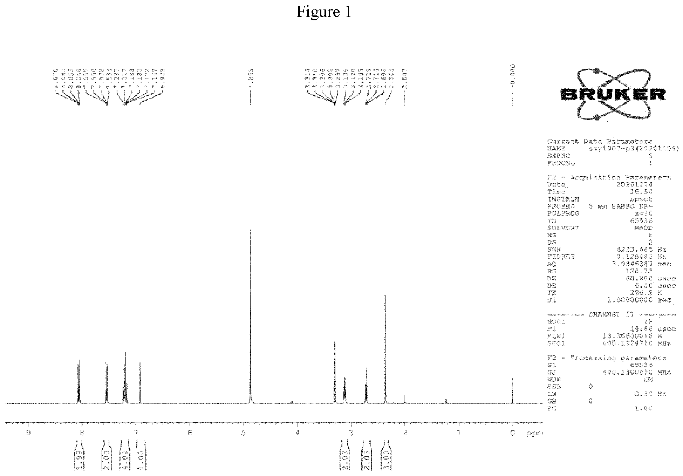

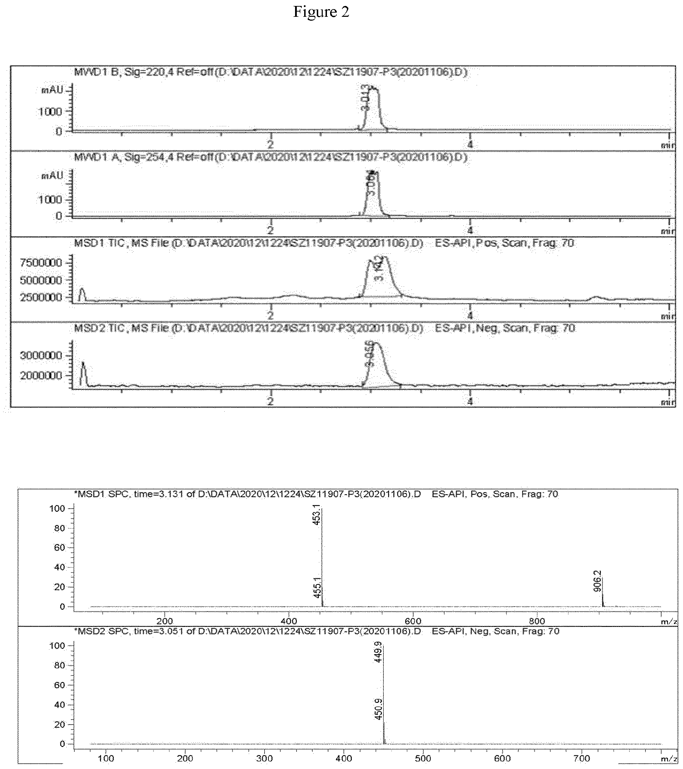

The results of structural confirmation are shown in Figures 1 and 2.

### Example 2. Preparation of different salt forms of SZY1907-P3

1. Preparation of citrate of SZY1907-P3:
   SZY1907-P3 was dissolved in water, 1 mol/L of sodium hydroxide was added dropwise until a solid was precipitated, followed by vacuum filtration, drying, weighing and dissolving 100 mg solid in methanol, to which 42 mg of citric acid was added and stirred for 30 minutes until clarification, rotary dried the methanol, to obtain 140 mg of the citrate.
2. Preparation of trans-butene dioic acid salt of SZY1907-P3:
   SZY1907-P3 was dissolved in water, 1 mol/L of sodium hydroxide was added dropwise until a solid was precipitated, followed by vacuum filtration, drying, weighing and dissolving 100 mg solid in methanol, to which 26 mg of fumaric acid was added and stirred for 30 minutes until clarification, rotary dried the methanol, to obtain 110 mg of the fumaric acid salt.
3. Preparation of salicylate of SZY1907-P3:
   SZY1907-P3 was dissolved in water, 1 mol/L of sodium hydroxide was added dropwise until a solid was precipitated, followed by vacuum filtration, drying, weighing and dissolving 100 mg solid in methanol, to which 31 mg salicylic acid was added and stirred for 30 minutes until clarification, rotary dried the methanol, to obtain 120 mg of the salicylate.
4. Preparation of L-tartrate of SZY1907-P3:
   SZY1907-P3 was dissolved in water, 1 mol/L of sodium hydroxide was added dropwise until a solid was precipitated, followed by vacuum filtration, drying, weighing and dissolving 100 mg solid in methanol, to which 33 mg of L-tartaric acid was added and stirred for 30 minutes until clarification, rotary dried the methanol, to obtain 125 mg of the L-tartrate.

### Example 3. Preliminary study on solubility and stability of ZY1907-P3

In the initial solubility test of SZY1907-P3, it was found that the solubility of its hydrochloride was related to pH value, showing a certain pH dependence, so the solubility curves of its prototype and its salt form at different pH values were determined.

At present, the prototype, citrate, acetate, tartrate, and the like have been determined, and the specific data are shown in Table 1 below.

**Table 1**

| pH value | Solubility of prototype mg/mL | Solubility of citrate mg/mL | Solubility of acetate mg/mL | Solubility of lysinate mg/mL | Solubility of aspartate mg/mL | Solubility of phosphate mg/mL | Solubility of tartrate mg/mL | Solubility of fumarate mg/mL |
|---|---|---|---|---|---|---|---|---|
| 8 | 0.08 | 0.14 | 0.1 | 0.1 | 0.12 | 0.1 | 0.11 | 0.09 |
| 7 | 0.09 | 0.16 | 0.1 | 0.09 | 0.13 | 0.09 | 0.11 | 0.09 |
| 6 | 0.08 | 0.37 | 0.09 | 0.09 | 0.19 | 0.47 | 0.21 | 0.11 |
| 4 | 0.08 | 0.72 | 0.1 | 0.1 | 0.19 | 1.28 | 0.12 | 0.14 |
| 2 | 1.02 | 42.27 | 0.68 | 0.08 | 0.43 | 25.72 | 0.26 | 0.19 |
| 1 | 83.46 | 94.52 | 94.56 | 40.47 | 74.09 | 42.9 | 64.39 | 73.10 |
| | | Pka 3.13 | Pka 4.76 | Pka 2.16 | Pka3.90 | Pka2.12 | Pka 3.0 | Pka 3.03 |

It can be seen that the solubility of P3 prototype and the different salt forms thereof has strong pH dependence. When pH ≥ 4, the solubility is less than 1 mg/mL.

Meanwhile, the stability data of the prototype at different pH values were measured, and the results are as shown in Table 2 below.

**Table 2**

| No. | pH value | Solubility mg/mL | Purity at Day 0 | Purity at Day 1 | Purity at Day 2 | Purity at Day 5 |
|---|---|---|---|---|---|---|
| 1 | 5.5-6.0 | 0.1 | 99.85% | 99.32% | 99.67% | 99.63% |
| 2 | 6.5-7.0 | 0.09 | 99.88% | 100.00% | 99.74% | 99.87% |
| 3 | 7.0-7.5 | 0.1 | 99.81% | 100.00% | 99.70% | 99.84% |

It can be seen from Table 2 that there is no significant change in purity when stored for 5 days.

### Comparative Example 1. Preparation of compound SZY1907-P4

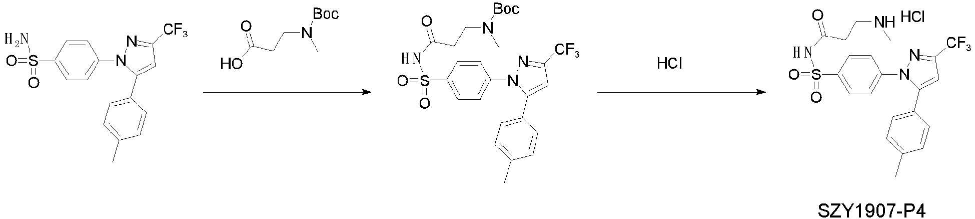

### Preparation of SZY1907-06:

Celecoxib (1.34 g, 3.5 mmol) and DCM (26 mL) was added to a three-neck flask, then N-methyl-Boc-β-alanine (4.9 mmol), EDCI (8.05 mmol), triethylamine (10.5 mmol), DMAP (1 mmol) were added successively; stirred at room temperature, and monitored by TLC until no more raw materials were detected.

Post-treatment: washing once with 3% diluted hydrochloric acid, washing once with saturated ammonium chloride, washing with saturated sodium chloride to a neutral condition, drying the organic phase with sodium sulfate, rotary drying to obtain a white solid; mixing the white solid and passing through a column, washing with dichloromethane/methanol and at 30:1 (v/v) the product was eluted; collecting and rotary drying to obtain 1.3 g of a white solid.

State: White solid.

### Preparation of SZY1907-P4:

SZY1907-04 (1.00 g, 1.77 mmol) was added to a three-neck flask, and then a solution of hydrogen chloride in ethyl acetate (3.8 mol/L, 30 mL) was added, stirred, and monitored by TLC until no more raw materials were detected.

Post-treatment: rotary drying to obtain a white solid; dissolving with methanol, adding 2 mL of triethylamine, mixing the sample and passing through a column, washing with dichloromethane/methanol and at 15:1 (v/v) the product was eluted, collecting the organic phase, adding 5 mL of hydrochloride in ethyl acetate , rotary drying to obtain 0.6 g of a white solid.

State: White solid.

¹HNMR H₂O δ: 7.56-7.54 (d, J=8.0 Hz, 2H), 6.87-6.84 (d, J=7.6 Hz, 2H),6.59-6.50 (m, 4H), 6.20 (s, 1H), 3.06-3.03 (m, 2H), 2.63-2.54 (m, 5H), 1.78 (m, 3H).

LC-MS: m/z = 467.5(M+1).

### Example 4. Ocular pharmacokinetic tests of compound SZY1907-P3 and compound SZY1907-P4

**Object of the test:** To compare the content of the active compound celecoxib in each eye tissue to screen out lead compound with excellent pharmacokinetic characteristics after the ocular administration of SZY1907-P3, SZY1907-P4 and celecoxib in New Zealand rabbits.

### Experimental materials:

Test product:
SZY1907-P3: white powder, molecular formula: C₂₀H₂₀ClF₃N₄O₃S, molecular weight: 488.91, batch number: 20201106, purity: 99.85%, storage conditions: 2-8 °C, valid until May 05, 2021.
SZY1907-P4: white solid, molecular formula: C₂₁H₂₂ClF₃N₄O₃S, molecular weight: 502.9, batch number: 20200801, purity: 98.99%, storage conditions: 2-8 °C, valid until July 31, 2021.
Celecoxib: white powdery solid, molecular formula: C₁₇H₁₄F₃N₃O₂S, molecular weight: 381.37, batch number: 20190701, retest purity: 99.87%, storage conditions: 2-8 °C, protected from light, valid until June 27, 2021.
Solvent: the formulation of SZY1907-P3/P4 eye drops are shown in Table 3.

Table 3 shows the solvent components and functions thereof.

**Table 3. Solvent components (w/v) and functions**

| Components | Concentration (w/v) | Function |
|---|---|---|
| Tween-80 | 0.05% | co-solvent |
| Polyoxyethylene 40 hydrogenated castor oil | 0.45% | co-solvent |
| Sodium chloride | 0.9% | osmotic regulator |
| Benzalkonium chloride | 0.01% | preservative |

Solvent of celecoxib: 2% of polyethylene glycol 400 + 98% of 7.5% methylated β-cyclodextrin aqueous solution.

Tool drugs and main reagents:
Methanol (chromatographic pure): German Merck.
Formic acid (chromatographic pure): Aladdin.
Methylated-β-cyclodextrin: MedChem Express.
Polyethylene glycol 400: Solarbio Life Sciences.
Distilled water: Guangzhou Watsons Food & Beverage Co., Ltd.
Tween-80: VETEC.
Polyoxyethylene 40 hydrogenated castor oil: Croda.
Benzalkonium chloride solution (concentration of 80%): Sinopharm Chemical Reagent Co., Ltd.
Sodium chloride: VETEC INC.
The solution of Diazepam in methanol: provided by the New Drug Evaluation Center of Hebei Yiling Pharmaceutical Research Institute Co., Ltd.

### Experiment system:

Animal lineage: New Zealand rabbit.
Animal Level: Normal Level.
Gender and number of animals: 12 males were purchased, 9 of them were selected for this experiment, and the remaining 3 were used for taking blank tissue samples.
Age of animals at time of purchase: 3-5 months old.
Animal weight at time of purchase: 1.5-2.0 kg.
Adaptive rearing: Newly received animals were adaptively reared for 3-5 days. During this time, the animal drinking water, ingestion and health status were observed, as well as whether there were symptoms of disease and death.
Identification: Numbered with ear marker pen.

### Basis for experimental design

Adopted standard: Technical guidelines for non-clinical pharmacokinetic research of drugs issued by the State Food and Drug Administration.

### Dosage and grouping

Grouping: 9 male New Zealand rabbits were randomly divided into 3 groups of 3 animals each.

Dosage: According to the formulation of Guangzhou Zhongshan Ophthalmic Center, the concentration of SZY1907-P3/P4 in eye drops is 0.1%. Thus, the dose of SZY1907-P3/P4 was set to 0.100 mg/New Zealand rabbit and the dose of celecoxib was 0.075 mg/New Zealand rabbit (close to the equimolar dose of SZY1907-P3/P4). The specific grouping and dosage are shown in Table 4.

**Table 4. Dosage of SZY1907-P3, SZY1907-P4 and celecoxib**

| Group | Number of animals | Dosage (mg/ New Zealand rabbit) | | |
|---|---|---|---|---|
| | ♂ | SZY1907-P3 | SZY1907-P4 | Celecoxib |
| SZY1907-P3 group | 3 | 0.100 | 0 | 0 |
| SZY1907-P4 group | 3 | 0 | 0.100 | 0 |
| Celecoxib group | 3 | 0 | 0 | 0.075 |

The administration method was consistent with the clinical method, which was, ophthalmic administration, and the rabbit left and right eyes were simultaneously administrated.

Preparation and storage of test product (the preparation volume in the following operations can be adjusted according to actual needs).

0.1% of SZY1907-P3 eye drops: 0.01000 g of SZY1907-P3 was weighed, 0.00500 g of Tween-80 and 0.04500 g of polyoxyethylene 40 hydrogenated castor oil were added, and stirred with a glass rod until the solution was in an emulsion shape. 5 mL of distilled water was added slowly while stirring, dissolved for later use. 0.09000 g of sodium chloride and 0.00125 g of 80% benzalkonium chloride were weighed, 2 mL of distilled water was added, and stirred to dissolve. The two solutions was combined and adjusted the volume to 10 mL. The pH was adjusted to a range of 5.5 to 6.5 with 1 mol/L of NaOH solution.

0.1% of SZY1907-P4 eye drops: 0.01000 g of SZY1907-P4 was weighed, 0.00500 g of Tween-80 and 0.04500 g of polyoxyethylene 40 hydrogenated castor oil were added, and stirred with a glass rod until the solution was in an emulsion shape. 5 mL of distilled water was added slowly while stirring, dissolved for later use. 0.09000 g of sodium chloride and 0.00125 g of 80% benzalkonium chloride was weighed, 2 mL of distilled water was added, and stirred to dissolve. The two solutions were combined and adjusted the volume to 10 mL. The pH was adjusted to a range of 5.5 to 6.5 with 1 mol/L of NaOH solution.

0.075% of celecoxib eye drops: 0.00750 g of celecoxib was added into a 1.5 mL of centrifuge tube, then 200 µL of polyethylene glycol 400 was added, centrifuged at 12000 rpm for 60 s, then transferred to a 10 mL of volumetric flask, 7.5% of methylated-β-cyclodextrin aqueous solution was added to adjust the volume, shaking until the solution was clear and transparent to obtain the eye drops.

Administration of the test product: administering to the rabbit left and right eyes at the same time with the dosage of 50 µL per eye. Precise administration with a pipette: gently pulling the rabbit eyelid low down as a cup-shape, using a pipette to accurately take 50 µL of the eye drops and administering it dropwise into the eyelid, then passively closing the rabbit eyes for 10 seconds.

Observation indicators, time and content: sample collection: collecting aqueous humor, cornea and conjunctiva at 1 h after administration. Collection of aqueous humor: for sacrificed animals, drawing approximately 200 µL of aqueous humor with a 1 mL syringe at the junction between the pupil and iris (note that the tangent plane of the syringe needle was facing down when aqueous humor was drawn to prevent aqueous humor from spraying). Collection of cornea: fixing the eyeball with forceps, cutting the cornea and iris with curved scissors, and cutting the cornea along the junction between them. Washing the removed cornea with ultrapure water, sopping up the surface liquid with filter paper, weighing precisely and cryopreservation. Collection of conjunctiva: Clamping the upper and lower eyelid conjunctiva with forceps and cutting it with scissors. Cleaning the removed conjunctiva with ultrapure water, sopping up the surface liquid with filter paper, weighing precisely and cryopreservation.

Treatment of tissue samples: preparation of cornea/conjunctiva homogenate: first shearing the corneas/conjunctivas of the left and right eyes into small pieces with small scissors, then adding 50% methanol aqueous solution (mass to volume ratio of 1:10), grinding with a grinder (the procedure was to grind 4 times in cycles, grinding at 6500 rpm per cycle for 30 s and waiting for 20 s), centrifuging at 4000 rpm with a low-speed centrifuge for 10 min, aspirating the supernatant, and cryopreservation for measurement.

Pretreatment before sample detection: aqueous humor sample: taking 50 µL of aqueous humor, adding 200 µL of internal standard working solution (100 ng/mL diazepam in methanol solution), vortex mixing for 5 min, centrifuging at 12000 rpm with a high-speed centrifuge for 10 min; aspirating the supernatant and loading into the autosampler vial for measurement.

Cornea and conjunctival samples: taking 50 µL of homogenate supernatant, adding 200 µL of internal standard working solution (100 ng/mL diazepam in methanol solution), vortex mixing for 5 min, centrifuging at 12000 rpm with a high-speed centrifuge for 10 min; aspirating the supernatant and loading into the autosampler vial for measurement.

Instrumentation: Liquid chromatography-mass spectrometer: Waters Xevo TQD/PDA ACQUITY UPLC Liquid chromatography-mass spectrometer equipped with electrospray ionization source (ESI source) (Waters).

Data system: Masslynx V4.1 (Waters).

Data processing: Calculated by the ratio of peak area of the sample to the internal standard. The weighted (W=1/X 2) least squares method was used for regression calculation, the linear regression equation was obtained, and the drug concentration was calculated after substituting the ratio into the equation.

### Experimental results:

The average drug concentration of celecoxib in aqueous humor at 1 h after administration of 0.1% of SZY1907-P3/P4, and 0.075% of celecoxib respectively to the New Zealand rabbit eyes

| Group | Aqueous humor | | | |
|---|---|---|---|---|
| | No. | Concentration (ng/mL) | Mean | SD |
| SZY1907-P3 group | 1 | 43.8 | 151.8 | 118.7 |
| | 2 | 132.8 | | |
| | 3 | 278.9 | | |
| SZY1907-P4 group | 4 | ND | - | - |
| | 5 | ND | | |
| | 6 | ND | | |
| Celecoxib group | 7 | 56.8 | 36.9 | 17.6 |
| | 8 | 23.0 | | |
| | 9 | 31.1 | | |

| | | | | |
|---|---|---|---|---|
| "ND" represents not detected, and "-" represents cannot be calculated. | | | | |

The average drug concentration of celecoxib in cornea at 1 h after administration of 0.1% of SZY1907-P3/P4, and 0.075% of celecoxib respectively to the New Zealand rabbit eyes

| Group | Cornea | | | |
|---|---|---|---|---|
| | No. | Concentration (ng/g) | Mean | SD |
| SZY1907-P3 group | 1 | 1072.1 | 1385.9 | 274.6 |
| | 2 | 1503.4 | | |
| | 3 | 1582.1 | | |
| SZY1907-P4 group | 4 | 14.3 | 35.6 | 20.2 |
| | 5 | 54.6 | | |
| | 6 | 38.0 | | |
| Celecoxib group | 7 | 3668.3 | 1980.0 | 1467.7 |
| | 8 | 1008.3 | | |
| | 9 | 1263.5 | | |

| | | | | |
|---|---|---|---|---|
| "ND" represents not detected, and "-" represents cannot be calculated. | | | | |

The average drug concentration of celecoxib in conjunctiva at 1 h after administration of 0.1% of SZY1907-P3/P4, and 0.075% of celecoxib respectively to the New Zealand rabbit eyes

| Group | Conjunctiva | | | |
|---|---|---|---|---|
| | No. | Concentration (ng/g) | Mean | SD |
| SZY1907-P3 group | 1 | 762.9 | 548.4 | 229.1 |
| | 2 | 307.1 | | |
| | 3 | 575.3 | | |
| SZY1907-P4 group | 4 | ND | - | - |
| | 5 | ND | | |
| | 6 | ND | | |
| Celecoxib group | 7 | 346.2 | 161.0 | 162.0 |
| | 8 | 45.5 | | |
| | 9 | 91.2 | | |

| | | | | |
|---|---|---|---|---|
| "ND" represents not detected, and "-" represents cannot be calculated. | | | | |

From the above results, it can be seen that celecoxib can be detected in ocular tissues and is distributed in cornea, conjunctiva and aqueous humor, and the celecoxib achieves a relatively high drug concentration and thus the compound of Formula I is an excellent celecoxib prodrug. Moreover, the distribution of control compound SZY1907-P4 is almost not detected, and the concentration of eye drops prepared from celecoxib in the cornea, conjunctiva and aqueous humor is also relatively low. The compound of Formula I SZY1907-P3 has a remarkable absorption advantage, the drug concentration in the ocular tissues is remarkably higher than those of the celecoxib group and control group SZY1907-P4, and thus the compound can be prepared into liquid preparations, such as eye drops and injections, for inflammatory reactions.

## Claims

1. Compound of Formula I or a pharmaceutically acceptable salt thereof:

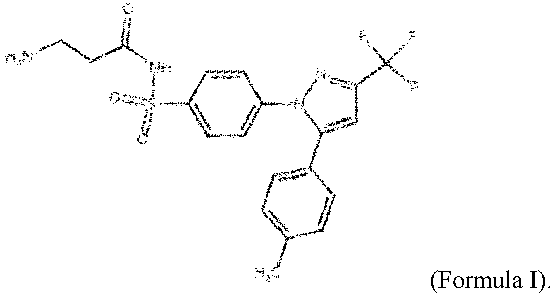

2. The compound or pharmaceutically acceptable salt thereof according to claim 1, wherein the pharmaceutically acceptable salt of the compound of Formula I include citrate, trans-butene dioic acid salt, salicylate, L-tartrate, fumarate, sodium salt, potassium salt, calcium salt, hydrochloride, acetate, nitrate, sulfate, bisulfate, phosphate, hydrophosphate, acetate, oxalate, lactate, lysinate, and aspartate.

3. A method for the preparation of the compound of Formula I according to claim 1, comprising the following steps:
1) the compound of Formula II and the compound of Formula III are reacted in the presence of EDCI/ DMAP/ triethylamine, to obtain the compound of Formula IV; and 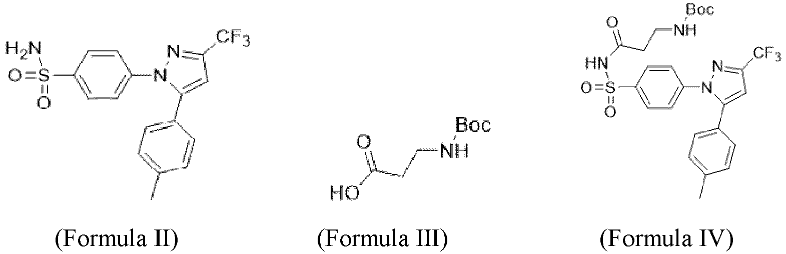
2) the compound of Formula IV is reacted with a solution of hydrogen chloride in ethyl acetate, to obtain the compound of Formula I.

4. The method according to claim 3, wherein in the step 1) the molar ratio of the compound of Formula II to the compound of Formula III is 1:1.4;
in the step 1) the molar ratio of the compound of Formula II to EDCI, DMAP and triethylamine is2.3: 0.31: 3, respectively;
in the step 1) the reaction is performed in a solvent, wherein the solvent is DCM; and
in the step 1) the reaction is performed under stirring conditions at room temperature and monitoring by TLC until there is no more raw material.

5. The method according to claim 3 or 4, wherein in the step 1) the method further comprises the following post-treatment steps after the reaction is finished: washing once with 3% diluted hydrochloric acid, washing once with saturated ammonium chloride, washing with saturated sodium chloride to a neutral condition, drying the organic phase with sodium sulfate, rotary drying to obtain a white solid; dissolving the white solid in ethanol, adding an equivalent amount of water, heating and refluxing until the white solid being completely dissolved, slowly cooling to precipitate a product, vacuum filtration and drying to obtain the compound of Formula IV.

6. The method according to any one of claims 3 to 5, wherein in the step 2) the molar ratio of the compound of Formula IV to hydrogen chloride is 0.05:1.71; and
in the step 2) the reaction condition of the reaction is stirring at room temperature; a solid is precipitated gradually during the reaction process, and the reaction is monitored by TLC until there is no more raw material.

7. The method according to any one of claims 3 to 6, wherein in the step 2) the method further comprises the following post-treatment steps after the reaction is finished: rotary drying, drip washing the solid with ethyl acetate, collecting the filter cake and rotary drying to a constant weight to obtain the compound of Formula I.

8. Use of the compound of Formula I or a pharmaceutically acceptable salt thereof in the preparation of a medicament for the prevention and/or treatment of inflammation of an ocular tissue.

9. A medicament or a pharmaceutical composition for the prevention and/or treatment of inflammation of an ocular tissue, comprising the compound of Formula I or a pharmaceutically acceptable salt thereof as the active ingredient.

10. An eye drops for the prevention and/or treatment of inflammation of an ocular tissue, containing the following constituents per 100 mL of the eye drops: 0.1 g of the compound of Formula I, 0.05 g of Tween-80, 0.45 g of polyoxyethylene 40 hydrogenated castor oil, 0.9 g of sodium chloride, 0.01 g of benzalkonium chloride, and the balance being distilled water; wherein the pH value of the eye drops is 5.5-6.5.
